# FASCICULE DE BREVET EUROPEEN

(11) **EP 4 061 271 B1**
(45) Date de publication et mention de la délivrance du brevet: **22.01.2025**
(21) Numéro de dépôt: 20823906.1
(22) Date de dépôt: 17.11.2020
(51) Int. Cl.: A61B 34/20, A61B 90/00, A61B 34/30

(54) **MÉTHODE DE NAVIGATION POUR POSITIONNER UN ROBOT MÉDICAL**
NAVIGATIONSVERFAHREN ZUM POSITIONIEREN EINES MEDIZINISCHEN ROBOTERS
NAVIGATION METHOD FOR POSITIONING A MEDICAL ROBOT

(30) Priorité: 19.11.2019 FR 1912907
(43) Date de publication de la demande: 28.09.2022
(73) Titulaire: Quantum Surgical, 34000 Montpellier (FR)
(72) Inventeur: NAHUM, Bertin, 34170 Castelnau-le-Lez (FR); BADANO, Fernand, 69006 Lyon (FR); BLONDEL, Lucien, 34070 Montpellier (FR)
(74) Mandataire: Ipside
(86) Numéro de dépôt international: PCT/FR2020/052100
(87) Numéro de publication internationale: WO 2021/099729

(56) Documents cités:
- EP-A1- 2 944 285
- EP-A1- 3 501 443
- WO-A1-2016/014718
- US-A1- 2008 285 725
- US-A1- 2019 038 362

## Description

### Domaine de l'invention

La présente invention appartient au domaine des interventions médicales mini-invasives et non-invasives assistées par un robot médical. L'invention concerne notamment un système de navigation optique permettant de déterminer la position d'une anatomie d'intérêt d'un patient afin de positionner de manière optimale un instrument médical fixé à une extrémité d'un bras articulé du robot médical. En particulier, l'invention permet de déterminer la position de l'anatomie d'intérêt même lorsqu'un obstacle empêche d'obtenir une ligne de mire directe entre un marqueur optique situé au niveau de l'anatomie d'intérêt et un capteur optique du système de navigation. L'invention concerne également un procédé pour déterminer la position d'une anatomie d'intérêt d'un patient.

### Etat de la technique

De nombreuses interventions médicales, telles que les interventions médicales mini-invasives ou non-invasives, nécessitent de positionner ou de déplacer de manière très précise un instrument médical (par exemple une aiguille, un cathéter, une électrode, un générateur d'ultrasons, un foret de perçage, etc.) par rapport à une anatomie d'intérêt d'un patient (par exemple un foie, un poumon, un rein, une vertèbre etc.). Le praticien qui effectue ce type d'intervention médicale peut être assisté par un robot médical. Dans ce cas, le robot médical positionne, maintien et/ou guide un instrument médical par rapport à une anatomie d'intérêt d'un patient grâce à un système de navigation. L'instrument médical est par exemple fixé à une extrémité d'un bras articulé du robot médical. Le système de navigation permet de déterminer la position de l'instrument médical et la position de l'anatomie d'intérêt. Les informations sur les positions respectives de l'instrument médical et de l'anatomie d'intérêt l'une par rapport à l'autre permettent alors au robot médical de configurer son bras articulé de telle sorte que l'instrument médical soit positionné de manière optimale par rapport à l'anatomie d'intérêt.

Il existe différents systèmes de navigation. Les systèmes de navigation électromagnétique présentent l'inconvénient d'être sensibles aux interférences et aux distorsions du champ électromagnétique en présence de matériaux métalliques (tels que les moteurs d'un robot médical). Les systèmes de navigation optique présentent quant à eux l'inconvénient de ne plus fonctionner lorsque la ligne de mire (« line of sight » dans la littérature anglo-saxonne) entre un marqueur positionné au niveau de l'anatomie d'intérêt et un capteur optique du système de navigation est coupée par un obstacle (c'est par exemple le cas lorsque le praticien s'interpose entre ledit marqueur et ledit capteur optique).

Il n'est généralement pas suffisant de déterminer la position de l'anatomie d'intérêt à un instant favorable où une ligne de mire directe est disponible car l'anatomie d'intérêt du patient peut être en mouvement, par exemple à cause des mouvements de respiration du patient ou du déplacement de l'anatomie du fait du praticien. Il convient donc de pouvoir suivre la position de l'anatomie d'intérêt au cours du temps à l'aide du système de navigation, et ce même pendant des périodes où la ligne de mire entre un marqueur positionné au niveau de l'anatomie d'intérêt et un capteur optique du système de navigation est coupée par un obstacle.

Plusieurs solutions de l'art antérieur consistent à optimiser le positionnement des marqueurs et des capteurs optiques utilisés afin de réduire le risque que la ligne de mire soit coupée par un obstacle. Ces solutions sont généralement complexes et ne permettent pas toujours de garantir le fonctionnement du système de navigation optique lorsque la ligne de mire est coupée par un obstacle.

La demande de brevet EP 3501443 A1 divulgue notamment un système comprenant un composant rotatif intégré dans un scialytique pour déplacer une caméra vers une position appropriée pour viser une anatomie d'intérêt d'un patient lorsqu'un obstacle empêche d'obtenir une ligne de mire directe.

Certaines solutions de l'art antérieur cherchent à combiner un système de navigation optique avec un autre système de navigation complémentaire (par exemple un système de navigation électromagnétique) qui permettrait de garantir le fonctionnement du système de navigation lorsque la ligne de mire est coupée par un obstacle. Ces solutions aboutissent toutefois à des systèmes complexes, chers, et dont la précision peut être affectée par la présence d'objets métalliques.

### Exposé de l'invention

La présente invention a pour objectif de remédier à tout ou partie des inconvénients de l'art antérieur, notamment ceux exposés ci-avant.

A cet effet, et selon un premier aspect, il est proposé par la présente invention un système de navigation optique pour déterminer la position d'une anatomie d'intérêt d'un patient. Le système comprend notamment une référence patient destinée à être positionnée sur le patient au niveau de l'anatomie d'intérêt, un dispositif de localisation et une unité de contrôle. Le dispositif de localisation comporte au moins deux capteurs optiques. La référence patient comporte au moins trois marqueurs optiques. Les positions respectives des marqueurs optiques de la référence patient les uns par rapport aux autres sont connues a priori par l'unité de contrôle. Le système de navigation optique comporte en outre un dispositif réfléchissant dont la position dans un référentiel du dispositif de localisation est déterminable par l'unité de contrôle. Quand une ligne de mire directe entre la référence patient et chaque capteur optique est disponible, les capteurs optiques sont configurés pour mesurer, pour chaque marqueur optique de la référence patient, une grandeur représentative de la position dudit marqueur optique dans le référentiel du dispositif de localisation à partir d'un rayonnement optique provenant dudit marqueur optique et présentant pour chaque capteur optique un trajet direct entre ledit marqueur optique et ledit capteur optique. Quand une ligne de mire directe entre la référence patient et un capteur optique est coupée par un obstacle, les capteurs optiques sont configurés pour mesurer, pour chaque marqueur optique de la référence patient, une grandeur représentative de la position dudit marqueur optique dans le référentiel du dispositif de localisation à partir d'un rayonnement optique provenant dudit marqueur optique et présentant un trajet réfléchi par le dispositif réfléchissant vers chaque capteur optique. L'unité de contrôle est configurée pour déterminer, à partir des mesures réalisées par les capteurs optiques, la position de la référence patient dans le référentiel du dispositif de localisation, et pour en déduire la position de l'anatomie d'intérêt dans ledit référentiel.

Dans la présente demande, on entend par « rayonnement optique » un rayonnement électromagnétique compris dans une plage de longueur d'onde allant de 100 nm (cent nanomètres) à 1 mm (un millimètre). Ainsi, le rayonnement infrarouge, le rayonnement de la lumière visible et le rayonnement ultraviolet sont des rayonnements optiques. Le terme « rayon optique » est parfois employé pour définir un trajet particulier emprunté par un rayonnement optique.

Dans la présente demande, le terme « position » représente la position et l'orientation dans les trois dimensions d'un référentiel spatial (cela correspond au terme « pose » dans la littérature anglo-saxonne).

Par l'expression « la position du dispositif réfléchissant est déterminable par l'unité de contrôle », on entend que la position du dispositif réfléchissant est connue a priori par l'unité de contrôle (par exemple la position du dispositif réfléchissant est sauvegardée dans une mémoire de l'unité de contrôle), ou bien qu'elle peut être déterminée par l'unité de contrôle (par exemple à l'aide de marqueurs optiques disposés sur le dispositif réfléchissant).

Avec de telles dispositions, le système de navigation optique selon l'invention est capable de déterminer la position de l'anatomie d'intérêt du patient même lorsque la ligne de mire est coupée par un obstacle (par exemple par un praticien qui devra réaliser une intervention médicale sur l'anatomie d'intérêt, ou par le robot médical qui assiste ledit praticien).

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, le dispositif réfléchissant comporte au moins trois marqueurs optiques. Les positions respectives des marqueurs optiques du dispositif réfléchissant les uns par rapport aux autres sont connues a priori par l'unité de contrôle.

Dans des modes particuliers de réalisation, pendant une période où une ligne de mire directe entre la référence patient et chaque capteur optique est disponible, l'unité de contrôle est configurée pour estimer un mouvement suivi par la référence patient dans le référentiel du dispositif de localisation pendant un cycle de respiration du patient. Ensuite, à un instant où une ligne de mire directe entre la référence patient et un capteur optique n'est plus disponible, l'unité de contrôle est configurée pour déterminer la position de la référence patient en fonction d'une part des mesures réalisées par les capteurs optiques à partir des rayonnements optiques provenant des marqueurs optiques de la référence patient et réfléchis par le dispositif réfléchissant, et en fonction d'autre part du mouvement estimé de la référence patient.

Dans des modes particuliers de réalisation, la référence patient comporte en outre au moins trois marqueurs radio-opaques. Les positions respectives des marqueurs radio-opaques les uns par rapport aux autres sont connues a priori par l'unité de contrôle.

Dans des modes particuliers de réalisation, la position de l'anatomie d'intérêt dans le référentiel du dispositif de localisation est déterminée en fonction de la position de la référence patient dans ledit référentiel et en fonction d'une image médicale de l'anatomie d'intérêt du patient sur laquelle sont visibles les marqueurs radio-opaques de la référence patient.

Dans des modes particuliers de réalisation, la position de l'anatomie d'intérêt dans le référentiel du dispositif de localisation est déterminée en outre en fonction d'un modèle biomécanique de l'anatomie d'intérêt.

Dans des modes particuliers de réalisation, le système de navigation optique comporte trois dispositifs réfléchissants orthogonaux deux à deux.

Dans des modes particuliers de réalisation, le système de navigation optique comporte en outre une référence robot destinée à être positionnée au niveau d'une extrémité distale d'un bras articulé d'un robot médical. La référence robot comporte au moins trois marqueurs optiques. Les positions respectives des marqueurs optiques les uns par rapport aux autres sont connues a priori par l'unité de contrôle. Quand une ligne de mire directe entre la référence robot et chaque capteur optique est disponible, les capteurs optiques du dispositif de localisation sont configurés pour mesurer, pour chaque marqueur optique de la référence robot, une grandeur représentative de la position dudit marqueur optique dans le référentiel du dispositif de localisation à partir d'un rayonnement optique provenant dudit marqueur optique et présentant pour chaque capteur optique un trajet direct entre ledit marqueur optique et ledit capteur optique. Quand une ligne de mire directe entre la référence robot et un capteur optique est coupée par un obstacle, les capteurs optiques sont configurés pour mesurer, pour chaque marqueur optique de la référence robot, une grandeur représentative de la position dudit marqueur optique dans le référentiel du dispositif de localisation à partir d'un rayonnement optique provenant dudit marqueur optique et présentant un trajet réfléchi par le dispositif réfléchissant vers chaque capteur optique. L'unité de contrôle est configurée pour déterminer la position de la référence robot dans le référentiel du dispositif de localisation à partir des mesures ainsi réalisées par les capteurs optiques.

Dans des modes particuliers de réalisation, le système de navigation optique comporte en outre un robot médical comportant un bras articulé. La référence robot est positionnée à une extrémité distale du bras articulé. Le robot médical comporte en outre des codeurs d'articulation du bras articulé permettant de déterminer à tout instant la position de la référence robot dans un référentiel du robot médical. Le robot médical est configuré pour transmettre à l'unité de contrôle la position de la référence robot dans le référentiel du robot. L'unité de contrôle est configurée pour en déduire la position d'un instrument médical fixé à l'extrémité distale du bras articulé du robot médical par rapport à l'anatomie d'intérêt du patient.

Dans des modes particuliers de réalisation, les marqueurs optiques de la référence patient et/ou de la référence robot sont des marqueurs actifs et le rayonnement optique provenant d'un marqueur optique est un rayonnement infrarouge généré par ledit marqueur optique.

Dans des modes particuliers de réalisation, les marqueurs optiques de la référence patient et/ou de la référence robot sont des marqueurs passifs et le rayonnement optique provenant d'un marqueur optique est un rayonnement infrarouge généré par le dispositif de localisation et réfléchi par ledit marqueur optique.

Selon un deuxième aspect, la présente invention concerne un procédé pour déterminer la position d'une anatomie d'intérêt d'un patient lors d'une intervention chirurgicale. Le procédé est mis en oeuvre par un système de navigation optique qui comprend une référence patient destinée à être positionnée sur le patient au niveau de l'anatomie d'intérêt ainsi qu'un dispositif de localisation. Les positions respectives des marqueurs optiques les uns par rapport aux autres sont connues a priori. Le dispositif de localisation comporte au moins deux capteurs optiques. La référence patient comporte au moins trois marqueurs optiques. Le système de navigation optique comporte en outre un dispositif réfléchissant dont la position dans un référentiel du dispositif de localisation est connue. Le procédé comporte les étapes suivantes :
- quand une ligne de mire directe entre la référence patient et chaque capteur optique est disponible, une mesure, pour chaque marqueur optique de la référence patient, d'une grandeur représentative de la position dudit marqueur optique dans le référentiel du dispositif de localisation à partir d'un rayonnement optique provenant dudit marqueur optique et présentant pour chaque capteur optique un trajet direct entre ledit marqueur optique et ledit capteur optique,
- quand une ligne de mire directe entre la référence patient et un capteur optique est coupée par un obstacle, une mesure, pour chaque marqueur optique de la référence patient, d'une grandeur représentative de la position dudit marqueur optique dans le référentiel du dispositif de localisation à partir d'un rayonnement optique provenant dudit marqueur optique et présentant un trajet réfléchi par le dispositif réfléchissant vers chaque capteur optique,
- une détermination, à partir des mesures ainsi réalisées par les capteurs optiques, de la position de la référence patient dans le référentiel du dispositif de localisation,
- une détermination, dans ledit référentiel du dispositif de localisation, de la position de l'anatomie d'intérêt à partir de la position de la référence patient.

Il convient de noter que la position de l'anatomie d'intérêt du patient et le positionnement de l'instrument médical fixé à une extrémité d'un bras articulé du robot médical ont lieu préalablement à l'exécution du geste médical par le praticien. Ainsi, le procédé selon l'invention pour déterminer la position d'une anatomie d'intérêt d'un patient ne comporte pas d'étape de traitement thérapeutique ou chirurgical.

Dans des modes particuliers de mise en oeuvre, l'invention peut comporter en outre l'une ou plusieurs des caractéristiques suivantes, prises isolément ou selon toutes les combinaisons techniquement possibles.

Dans des modes particuliers de mise en oeuvre, le procédé comporte en outre les étapes suivantes :
- pendant une période où une ligne de mire directe entre la référence patient et chaque capteur optique est disponible, une estimation d'un mouvement suivi par la référence patient dans le référentiel du dispositif de localisation pendant un cycle de respiration du patient,
- à un instant où une ligne de mire directe entre la référence patient et un capteur optique n'est plus disponible, une détermination de la position de la référence patient en fonction d'une part des mesures réalisées par les capteurs optiques à partir des rayonnements optiques provenant des marqueurs optiques de la référence patient et réfléchis par le dispositif réfléchissant, et en fonction d'autre part du mouvement estimé de la référence patient.

Dans des modes particuliers de mise en oeuvre, la détermination de la position de l'anatomie d'intérêt dans le référentiel du dispositif de localisation est effectuée en outre à partir d'une image médicale de l'anatomie d'intérêt du patient sur laquelle sont visibles des marqueurs radio-opaques de la référence patient.

Dans des modes particuliers de mise en oeuvre, la détermination de la position de l'anatomie d'intérêt dans le référentiel du dispositif de localisation est effectuée en outre à partir d'un modèle biomécanique de l'anatomie d'intérêt.

Dans des modes particuliers de mise en oeuvre, le système de navigation optique comporte en outre une référence robot destinée à être positionnée au niveau d'une extrémité distale d'un bras articulé d'un robot médical. La référence robot comporte au moins trois marqueurs optiques, les positions respectives des marqueurs optiques les uns par rapport aux autres étant connues a priori. Le procédé comporte en outre les étapes suivantes :
- quand une ligne de mire directe entre la référence robot et chaque capteur optique est disponible, une mesure, pour chaque marqueur optique de la référence robot, d'une grandeur représentative de la position dudit marqueur optique dans le référentiel du dispositif de localisation, à partir d'un rayonnement optique provenant dudit marqueur optique et présentant pour chaque capteur optique un trajet direct entre ledit marqueur optique et ledit capteur optique,
- quand une ligne de mire directe entre la référence robot et un capteur optique est coupée par un obstacle, une mesure, pour chaque marqueur optique de la référence robot, d'une grandeur représentative de la position dudit marqueur optique dans le référentiel du dispositif de localisation à partir d'un rayonnement optique provenant dudit marqueur optique et présentant un trajet réfléchi par le dispositif réfléchissant vers chaque capteur optique,
- une détermination de la position de la référence robot dans le référentiel du dispositif de localisation à partir des mesures ainsi réalisées par les capteurs optiques.

Dans des modes particuliers de mise en oeuvre, le système de navigation optique comporte en outre un robot médical. Le robot médical comporte un bras articulé à une extrémité distale duquel la référence robot est positionnée. Le robot médical comporte aussi des codeurs d'articulation du bras articulé permettant de déterminer à tout instant la position de la référence robot dans un référentiel du robot médical. Le procédé comporte alors une étape de détermination de la position d'un instrument médical fixé à l'extrémité distale du bras articulé du robot médical par rapport à l'anatomie d'intérêt du patient.

### Présentation des figures

L'invention sera mieux comprise à la lecture de la description suivante, donnée à titre d'exemple nullement limitatif, et faite en se référant aux figures 1 à 10 qui représentent :
[Fig. 1] une représentation schématique d'un système de navigation optique selon l'invention lorsqu'une ligne de mire directe est disponible,
[Fig. 2] une représentation schématique d'un système de navigation optique selon l'invention lorsque la ligne de mire est coupée par un obstacle,
[Fig. 3] une représentation schématique d'une référence patient comportant trois marqueurs optiques et trois marqueurs radio-opaques,
[Fig. 4] une représentation schématique d'une référence robot comportant trois marqueurs optiques,
[Fig. 5] une représentation schématique d'un dispositif réfléchissant comportant quatre marqueurs optiques,
[Fig. 6] une représentation schématique de la détermination de la position d'un marqueur optique de la référence patient, lorsque la ligne de mire est coupée par un obstacle, en fonction des mesures réalisées par les capteurs optiques sur des rayons optiques réfléchis par le dispositif réfléchissant,
[Fig. 7] une représentation schématique des principales étapes d'un procédé pour déterminer la position d'une anatomie d'intérêt d'un patient,
[Fig. 8] une représentation schématique d'un mouvement estimé de la référence patient au cours d'un cycle de respiration du patient,
[Fig. 9a] une représentation schématique de la détermination de la position d'un marqueur optique de la référence patient, lorsque la ligne de mire est coupée par un obstacle, en fonction des mesures réalisées par les capteurs optiques et en fonction du mouvement estimé de la référence patient,
[Fig. 9b] une vue en coupe de la figure 9a,
[Fig. 10] une représentation schématique des principales étapes d'un mode particulier de mise en oeuvre d'un procédé pour déterminer la position d'une anatomie d'intérêt d'un patient.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'un mode de réalisation de l'invention

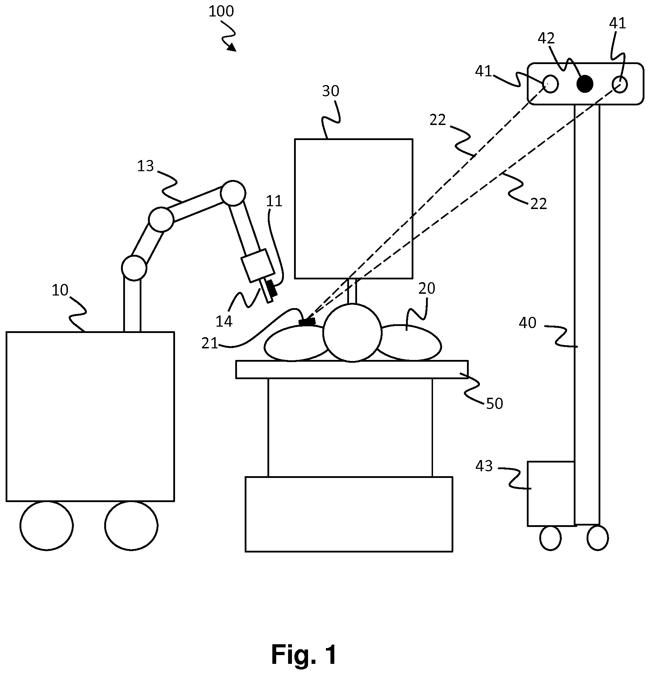

La figure 1 représente schématiquement un exemple de système de navigation optique 100 selon l'invention.

Dans l'exemple considéré et illustré à la figure 1, le système de navigation optique 100 comporte les éléments principaux suivants : un dispositif de localisation 40, une unité de contrôle 43, une référence patient 21 destinée à être positionnée au niveau d'une anatomie d'intérêt d'un patient 20 et un dispositif réfléchissant 30. Dans l'exemple illustré à la figure 1, le patient 20 est allongé sur une table 50 dans une salle d'intervention.

Le système de navigation optique 100 a pour objectif de déterminer la position de la référence patient 21 pour en déduire la position de l'anatomie d'intérêt dans un référentiel du dispositif de localisation 40. Dans ce but, l'unité de contrôle 43 est configurée pour mettre en oeuvre tout ou partie des étapes d'un procédé permettant de déterminer la position de la référence patient dans le référentiel du dispositif de localisation 40. L'unité de contrôle 43 comporte par exemple un ou plusieurs processeurs et une mémoire (disque dur magnétique, mémoire électronique, disque optique, etc.) dans laquelle est mémorisée un produit programme d'ordinateur, sous la forme d'un ensemble d'instructions de code de programme à exécuter pour mettre en oeuvre les différentes étapes d'un tel procédé. Alternativement ou en complément, l'unité de contrôle 43 comporte un ou des circuits logiques programmables (FPGA, PLD, etc.), et/ou un ou des circuits intégrés spécialisés (ASIC), et/ou un ensemble de composants électroniques discrets, etc., adaptés à mettre en oeuvre tout ou partie des étapes du procédé.

Tel qu'illustré sur la figure 1, le système de navigation optique 100 peut également comporter une référence robot 11 destinée à être positionnée au niveau d'une extrémité distale d'un bras articulé 13 d'un robot médical 10, par exemple sur un porte-instrument 14 fixé à ladite extrémité. L'unité de contrôle 43 peut alors être configurée pour déterminer également la position de la référence robot dans le référentiel du dispositif de localisation 40. Si la position d'un instrument médical fixé au niveau du porte-instrument 14 par rapport à la position de la référence robot 11 est connue par l'unité de contrôle 43, alors l'unité de contrôle 43 peut déterminer la position de l'instrument médical dans le référentiel du dispositif de localisation 40. Les positions respectives de l'instrument médical et de l'anatomie d'intérêt l'une par rapport à l'autre peuvent alors permettre au robot médical 10 de configurer son bras articulé de telle sorte que l'instrument médical soit positionné de manière optimale par rapport à l'anatomie d'intérêt.

L'unité de contrôle 43 peut être intégrée au dispositif de localisation 40, comme c'est le cas dans l'exemple illustré à la figure 1. L'unité de contrôle 43 peut également être une entité distincte du dispositif de localisation 40 ou encore être intégrée au robot médical 10. Si l'unité de contrôle 43 n'est pas intégrée au robot médical 10, l'unité de contrôle 43 peut être configurée pour transmettre au robot médical 10 des informations relatives à la position de l'anatomie d'intérêt et/ou à la position de l'instrument médical, de telle sorte que le robot médical 10 puisse configurer son bras articulé 13 de telle sorte que l'instrument médical soit positionné de manière optimale par rapport à l'anatomie d'intérêt. Cette transmission d'information peut par exemple être effectuée via des moyens de communication sans fil. Si l'unité de contrôle 43 n'est pas intégrée au dispositif de localisation 40, l'unité de contrôle 43 est configurée pour recevoir du dispositif de localisation 40 des informations relatives à la position de la référence patient 21 et/ou de la référence robot 11. La transmission d'information entre le dispositif de localisation 40 et l'unité de contrôle 43 peut par exemple être effectuée via des moyens de communication sans fil.

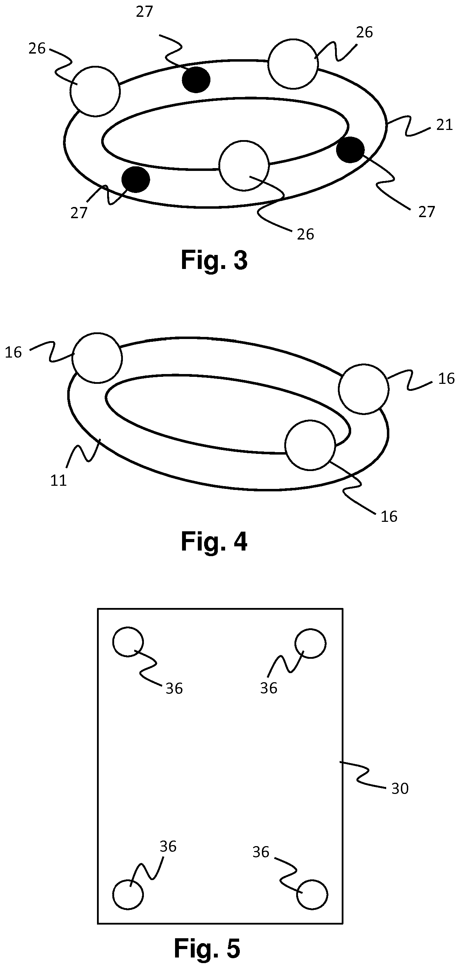

La figure 3 représente schématiquement la référence patient 21. La référence patient 21 comporte au moins trois marqueurs optiques 26, de telle sorte que la position de la référence patient 21 puisse être déterminée dans les trois dimensions spatiales du référentiel du dispositif de localisation 40. Les positions respectives des marqueurs optiques 26 de la référence patient 21 les uns par rapport aux autres sont connues a priori par l'unité de contrôle 43. Avantageusement, la forme géométrique de chaque marqueur optique 26 peut également être connue a priori par l'unité de contrôle 43. Dans l'exemple illustré à la figure 3, la référence patient 21 comporte trois marqueurs optiques 26 de forme sphérique. La forme sphérique permet d'optimiser la réflexion du rayonnement optique.

Les marqueurs optiques 26 peuvent être passifs ou actifs. Des marqueurs optiques passifs réfléchissent un rayonnement optique émis par un autre élément, comme par exemple le dispositif de localisation 40. Des marqueurs optiques passifs peuvent correspondre par exemple à des sphères réfléchissantes détectables par une caméra stéréoscopique infrarouge (c'est ce qui est utilisé par exemple dans les systèmes de navigation Polaris^{®} fabriqués par la société Northern Digital Inc.), ou à des motifs noir et blanc visibles par une caméra stéréoscopique (c'est ce qui est utilisé par exemple dans le système de navigation MicronTracker^{®} de la société ClaroNav). Des marqueurs optiques actifs émettent eux-mêmes un rayonnement optique, par exemple un rayonnement infrarouge, détectable par le dispositif de localisation 40.

La figure 4 représente schématiquement la référence robot 11. La référence robot 11 comporte au moins trois marqueurs optiques 16, de telle sorte que la position de la référence robot 11 puisse être déterminée dans les trois dimensions spatiales du référentiel du dispositif de localisation 40. Les positions respectives des marqueurs optiques 16 de la référence robot 11 les uns par rapport aux autres sont connues a priori par l'unité de contrôle 43. Avantageusement, la forme géométrique de chaque marqueur optique 16 peut également être connue a priori par l'unité de contrôle 43. Dans l'exemple illustré à la figure 4, la référence robot 11 comporte trois marqueurs optiques 16 de forme sphérique. Ce qui a été mentionné précédemment pour le type actif ou passif des marqueurs optiques 26 de la référence patient 21 est également vrai pour les marqueurs optiques 16 de la référence robot 11.

Les positions respectives des marqueurs optiques 16 de la référence robot 11 les uns par rapport aux autres diffèrent des positions respectives des marqueurs optiques 26 de la référence patient 21 les uns par rapport aux autres. De telles dispositions permettent au dispositif de localisation 40 de faire une distinction entre la référence patient 21 et la référence robot 11.

Tel qu'illustré sur la figure 1, le dispositif de localisation 40 comporte au moins deux capteurs optiques 41 correspondant par exemple à deux capteurs d'une caméra stéréoscopique fonctionnant dans le domaine des rayonnements infrarouges ou dans le domaine de la lumière visible. Dans la suite de la description, on considère à titre d'exemple nullement limitatif que les capteurs optiques 41 du dispositif de localisation 40 et les différents marqueurs optiques 16, 21 du système de navigation optique 100 sont conçus pour fonctionner avec un rayonnement optique de type infrarouge, c'est-à-dire un rayonnement électromagnétique dont la longueur d'onde varie entre 780 nm et 1 mm. Il convient cependant de noter que le système de navigation optique 100 selon l'invention pourrait également être conçu pour fonctionner dans domaine de la lumière visible (rayonnement électromagnétique dont la longueur d'onde varie entre 380 nm et 780 nm) ou dans le domaine de l'ultraviolet (rayonnement électromagnétique dont la longueur d'onde varie entre 10 nm et 380 nm).

De manière conventionnelle, et tel qu'illustré sur la figure 1, lorsqu'une ligne de mire directe est disponible entre la référence patient 21 et les capteurs optiques 41 du dispositif de localisation 40 (c'est-à-dire lorsqu'il n'y a pas d'obstacle entre la référence patient 21 et les capteurs optiques 41 du dispositif de localisation 40, ou autrement dit lorsque le rayonnement infrarouge peut suivre un trajet direct 22 en ligne droite entre chaque marqueur optique 26 de la référence patient 21 et chaque capteur optique 41 du dispositif de localisation 40), la position de chaque marqueur optique 26 peut être déterminée en fonction du temps de trajet d'un rayon infrarouge correspondant audit trajet direct 22 entre ledit marqueur optique 26 et un capteur optique 41 (la vitesse du rayonnement infrarouge étant connue puisqu'elle est égale à la vitesse de la lumière), et/ou en fonction d'un angle d'arrivée dudit rayon infrarouge au niveau dudit capteur optique 41.

Par exemple, lorsque les marqueurs optiques 26 utilisés dans le système de navigation optique 100 sont des marqueurs passifs, les capteurs optiques 41 peuvent être configurés pour émettre un rayonnement infrarouge. Ce rayonnement infrarouge est alors réfléchi par les différents marqueurs optiques 26 vers les capteurs optiques 41. Les capteurs optiques 41 sont configurés pour recevoir ce rayonnement infrarouge réfléchi. La distance entre un marqueur optique 26 et un capteur optique 41 est alors égal à la moitié du temps pris par un rayon infrarouge pour faire le trajet aller-retour entre ledit capteur optique 41 et ledit marqueur optique 26 multiplié par la vitesse de la lumière. En connaissant la distance entre chaque marqueur optique 26 et chaque capteur optique 41, et en connaissant a priori l'agencement des marqueurs optiques 26 les uns par rapport aux autres sur la référence patient 21, il est possible de déterminer la position de la référence patient 21 dans le référentiel du dispositif de localisation 40.

Selon un autre exemple, lorsque les marqueurs optiques 26 utilisés dans le système de navigation optique 100 sont des marqueurs actifs, chaque capteur optique 41 est configuré pour déterminer un angle d'arrivée au niveau dudit capteur optique 41 du rayonnement infrarouge qui est directement généré par un marqueur optique 26. En connaissant pour chaque marqueur optique 26 l'angle d'arrivée au niveau de chaque capteur optique 41, et en connaissant a priori l'agencement des marqueurs optiques 26 les uns par rapport aux autres sur la référence patient 21, il est possible de déterminer la position de la référence patient 21 dans le référentiel du dispositif de localisation 40.

De manière similaire, le dispositif de localisation 40 peut déterminer la position de la référence robot 11 dans le référentiel du dispositif de localisation 40 lorsqu'une ligne de mire directe est disponible entre la référence robot 11 et les capteurs optiques 41 du dispositif de localisation 40.

Il convient de pouvoir déterminer la position de l'anatomie d'intérêt du patient à partir de la position de la référence patient 21. Dans ce but, et tel qu'illustré sur la figure 3, la référence patient 21 peut comporter des marqueurs radio-opaques 27. Les positions respectives des marqueurs radio-opaques 27 les uns par rapport aux autres sont connues a priori par l'unité de contrôle 43. Avantageusement, la forme géométrique des marqueurs radio-opaques 27 peut également être connue a priori par l'unité de contrôle 43. De préférence, la référence patient 21 comporte au moins trois marqueurs radio-opaques 27. La position de l'anatomie d'intérêt dans le référentiel du dispositif de localisation 40 peut alors être déterminée en fonction de la position de la référence patient 21 dans ledit référentiel et en fonction d'une image médicale de l'anatomie d'intérêt du patient sur laquelle sont visibles les marqueurs radio-opaques 27 de la référence patient 21. L'image médicale donne en effet des informations sur la position de l'anatomie d'intérêt par rapport à la position de la référence patient 21. En connaissant la position de la référence patient 21 dans le référentiel du dispositif de localisation 40, on peut alors en déduire la position de l'anatomie d'intérêt dans ce référentiel.

Les marqueurs radio-opaques 27 peuvent être par exemple des billes en céramique ou des billes métalliques visibles dans une image médicale (par exemple tomodensitométrie par ordinateur, angiographie tridimensionnelle en rotation, imagerie par résonance magnétique, ultrasons, etc). Une image médicale du patient 20 est acquise avec la référence patient 21. Cette image médicale peut être recalée avec une autre image du même patient acquise préalablement et contenant des données de planification de l'intervention ou être utilisée directement pour planifier l'intervention. L'intervention planifiée peut être une ablation (par exemple par radiofréquence, micro-ondes, électroporation, laser, cryothérapie, ultrasons) d'une tumeur dans une anatomie d'intérêt (par exemple, le foie, le poumon ou le rein). L'intervention planifiée peut également être l'insertion d'un instrument médical dans le cerveau, dans la colonne vertébrale (par exemple pour une vertébroplastie et une cimentoplastie) ou encore dans une autre structure osseuse (par exemple, le genou). La planification comprend la détermination de la trajectoire devant être suivie par un instrument médical (par exemple une aiguille) entre un point d'entrée au niveau de la peau du patient et d'un point cible (dans la tumeur) dans l'anatomie d'intérêt. Une fois que la position de l'anatomie d'intérêt du patient est déterminée dans le référentiel du dispositif de localisation, il est possible de déduire à partir de ces données de planification la position que doit prendre l'instrument médical dans ce référentiel.

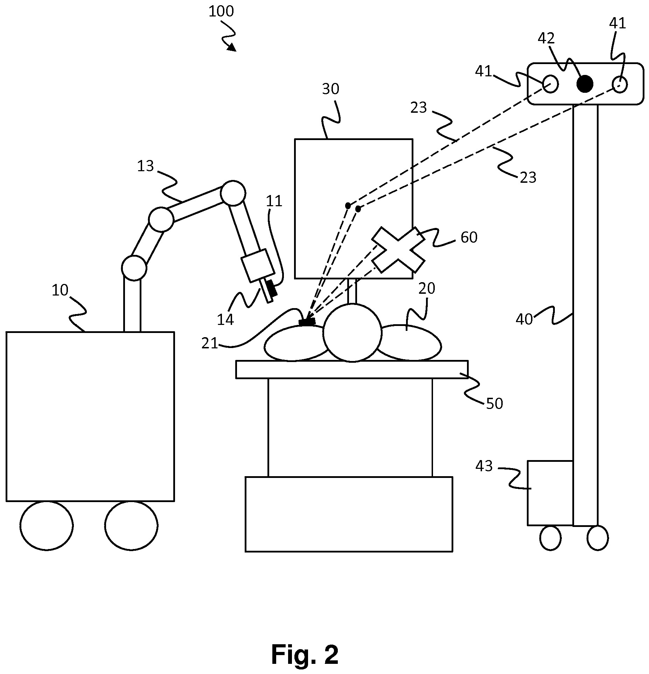

Tel qu'illustré sur la figure 2, un problème se pose pour la détermination de la position de la référence patient 21 lorsqu'un obstacle 60 coupe la ligne de mire entre la référence patient 21 et les capteurs 41 du dispositif de localisation 40 (et même si cela n'est pas illustré sur la figure 2, un problème similaire se pose pour la détermination de la position de la référence robot 11 lorsqu'un obstacle coupe la ligne de mire entre la référence robot 11 et les capteurs 41 du dispositif de localisation 40).

Pour pallier ce problème, la présente invention propose d'utiliser un dispositif réfléchissant 30 dont la position dans le référentiel du dispositif de localisation 40 est connue par l'unité de contrôle 43.

La position du dispositif réfléchissant 30 est par exemple connue a priori et mémorisée dans la mémoire de l'unité de contrôle 43. Dans une variante, et tel qu'illustré à la figure 5, le dispositif réfléchissant 30 comporte au moins trois marqueurs optiques 36. Les positions respectives des marqueurs optiques 36 les uns par rapport aux autres sont connues a priori par l'unité de contrôle 43. Avantageusement, la forme géométrique de chaque marqueur optique 36 peut également être connue a priori par l'unité de contrôle 43. La position du dispositif réfléchissant 30 peut alors être déterminée par l'unité de contrôle 43 à l'aide des capteurs optiques 41 du dispositif de localisation 40. Tel qu'illustré sur les figures 1 et 2, le dispositif réfléchissant 30 est par exemple fixé à la table 50 d'intervention, et la position du dispositif réfléchissant 30 n'est pas modifiée pendant la période de temps où l'anatomie d'intérêt du patient doit être déterminée.

Le dispositif réfléchissant 30 correspond par exemple à une plaque de verre sur laquelle est collée une fine feuille de métal (par exemple en aluminium ou en argent), elle-même recouverte d'une couche de cuivre ou de plomb. Alternativement, la plaque de verre peut être recouverte d'une fine feuille d'or. Le dispositif réfléchissant 30 peut être un miroir plat ou un miroir concave permettant de concentrer les rayons infrarouges. Dans l'exemple considéré, le dispositif réfléchissant 30 est un miroir plat de forme rectangulaire. Tel qu'illustré sur la figure 5, un marqueur optique 36 peut être positionné au niveau de chaque coin du rectangle formé par le dispositif réfléchissant 30.

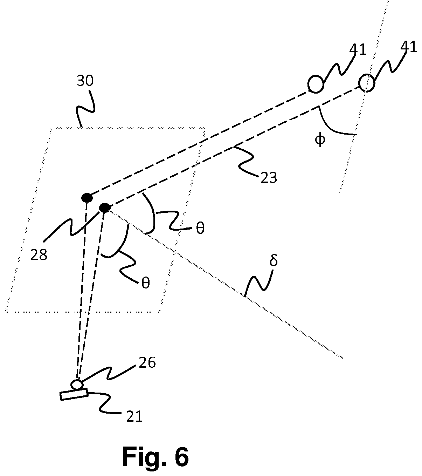

Tel qu'illustré sur la figure 2 et sur la figure 6, quand une ligne de mire directe entre la référence patient 21 et un capteur optique 41 est coupée par un obstacle 60, les capteurs optiques 41 sont configurés pour mesurer, pour chaque marqueur optique 26 de la référence patient 21, une grandeur représentative de la position dudit marqueur optique 26 dans le référentiel du dispositif de localisation 40 à partir d'un rayon infrarouge provenant dudit marqueur optique 26 en suivant un trajet réfléchi 23 par le dispositif réfléchissant 30 vers chaque capteur optique 41 (et non à partir d'un rayon infrarouge correspondant à un trajet direct 22 entre ledit marqueur optique 26 et ledit capteur optique 41). Par « une grandeur représentative de la position du marqueur » on entend par un exemple un temps de trajet du rayon infrarouge entre le capteur optique et le marqueur optique, ou bien un angle d'arrivée du rayon infrarouge au niveau du capteur optique 41.

Par exemple, et tel qu'illustré sur la figure 6, si l'on connaît pour chaque capteur optique 41 l'angle d'arrivée d'un rayon infrarouge 23 réfléchi par le dispositif réfléchissant 30 et provenant d'un marqueur optique 26 de la référence patient 21, et si l'on connaît la position du dispositif réfléchissant 30, il est alors possible de déterminer la position dudit marqueur optique 26. Il convient de noter que le rayon infrarouge 23 se réfléchit dans le dispositif réfléchissant 30 au niveau d'un point de réflexion 28 en formant un angle de réflexion θ par rapport à une droite δ perpendiculaire au dispositif réfléchissant 30 passant par le point de réflexion 28. Cet angle de réflexion θ est identique pour le rayon incident et pour le rayon réfléchi. Sur la figure 6, l'angle d'arrivée du rayon infrarouge 23 au niveau du capteur optique 41 dans un plan contenant le rayon infrarouge 23 et la droite δ correspond à l'angle Φ. Si l'angle d'arrivée Φ au niveau du capteur optique 41 et la position du dispositif réfléchissant 30 sont connues, il est possible de déterminer la valeur de l'angle de réflexion θ. Un angle de réflexion peut ainsi être déterminé pour le rayon infrarouge associé respectivement à chacun des deux capteurs optiques 41. La position du marqueur optique 26 correspond à l'intersection des deux rayons infrarouges.

Il convient de noter que la position du marqueur optique 26 pourrait également être déterminée à partir d'un capteur optique 41 en fonction d'une part de l'angle d'arrivée du rayon infrarouge 23 et en fonction d'autre part du temps de trajet du rayon infrarouge 23 entre le marqueur optique 26 et le capteur optique 41 (dans l'hypothèse où ce temps de trajet peut être déterminé).

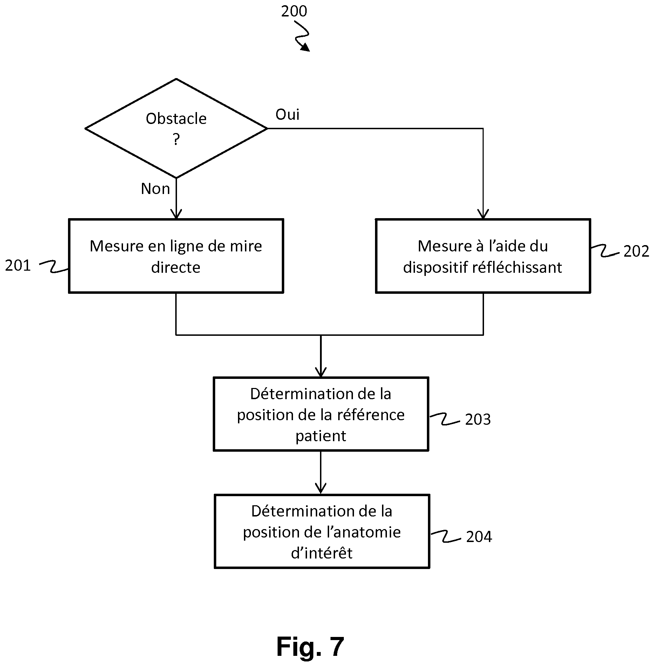

La figure 7 représente schématiquement les principales étapes d'un procédé mis en oeuvre par l'unité de contrôle 43 pour déterminer la position de l'anatomie d'intérêt du patient.

Le procédé 200 comporte une première étape dans laquelle il est détecté si un obstacle coupe une ligne de mire directe entre la référence patient 21 et les capteurs optiques 41 du dispositif de localisation 40.

Si une ligne de mire directe est disponible (pas d'obstacle), le procédé 200 comporte une étape 201 dans laquelle des mesures sont réalisées par les capteurs optiques 41 à partir de rayons infrarouges suivant des trajets directs 22 entre les marqueurs optiques 26 de la référence patient 21 et les capteurs optiques 41.

Si la ligne de mire est coupée (présence d'un obstacle), alors le procédé 200 comporte une étape 202 dans laquelle des mesures sont réalisées par les capteurs optiques 41 à partir de rayons infrarouges 23 suivant des trajets indirects comportant une réflexion sur le dispositif réfléchissant 30, entre les marqueurs optiques 26 de la référence patient 21 et les capteurs optiques 41.

A l'étape 203, la position de la référence patient 21 est déterminée à partir des positions des marqueurs optiques 26 déterminées à partir des mesures réalisées par les capteurs optiques 41.

A l'étape 204, la position de l'anatomie d'intérêt 204 est déterminée à partir de la position de la référence patient 21.

Les capteurs optiques 41 sont par exemple configurés pour travailler par défaut avec des rayons infrarouges 23 provenant directement d'un espace de travail dans lequel la référence patient 21 et/ou la référence robot 11 sont supposées se trouver. Si de tels rayons infrarouges ne sont pas détectés, alors l'unité de contrôle 43 configure les capteurs optiques 41 pour qu'ils travaillent à partir de rayons infrarouges 23 réfléchis par le dispositif réfléchissant 30.

Ainsi, il est possible de déterminer la position de l'anatomie d'intérêt du patient à tout moment, même lorsqu'une ligne de mire directe n'est pas disponible.

Ce qui vient d'être expliqué ci-dessus pour la détermination de la position de la référence patient 21 s'applique également à la détermination de la référence robot 11 lorsqu'un obstacle coupe la ligne de mire entre la référence robot 11 et les capteurs 41 du dispositif de localisation 40.

Dans des modes particuliers de réalisation, le robot médical 10 comporte en outre des codeurs d'articulation au niveau de son bras articulé 13 permettant de déterminer à tout instant la position de la référence robot 11 dans un référentiel du robot médical 10. Le robot médical 10 est configuré pour transmettre à l'unité de contrôle 43 la position de la référence robot 11 dans le référentiel du robot médical 10. L'unité de contrôle 43 est configurée pour en déduire la position d'un instrument médical fixé à l'extrémité distale du bras articulé 13 du robot médical 10 par rapport à l'anatomie d'intérêt du patient 20.

Dans ce but, il est par exemple envisageable, lors d'une étape préalable de mise en place, que le bras articulé 13 du robot médical 10 effectue un mouvement prédéfini pour faire prendre à la référence robot 11 différentes positions de calibrage réparties dans l'espace de travail commun du robot médical 10 et du système de navigation optique 100. Pour chaque position de calibrage prise par la référence robot 11, l'unité de contrôle 43 reçoit les informations de position d'une part par les codeurs d'articulation et d'autre part par les marqueurs optiques 16 de la référence robot 11. Le référentiel du robot médical 10 et le référentiel du dispositif de localisation 40 peuvent être recalés (mis en correspondance) par appariement des différentes positions de calibrage et calcul de recalage rigide point par point.

Lorsque le patient respire, l'anatomie d'intérêt du patient (et plus particulièrement une zone cible au sein de l'anatomie d'intérêt) suit les mouvements de la respiration. Il convient de pouvoir suivre la position de l'anatomie d'intérêt au cours du temps à l'aide du système de navigation optique 100, et ce même pendant des périodes où la ligne de mire entre un marqueur positionné au niveau de l'anatomie d'intérêt et un capteur optique du système de navigation est coupée par un obstacle.

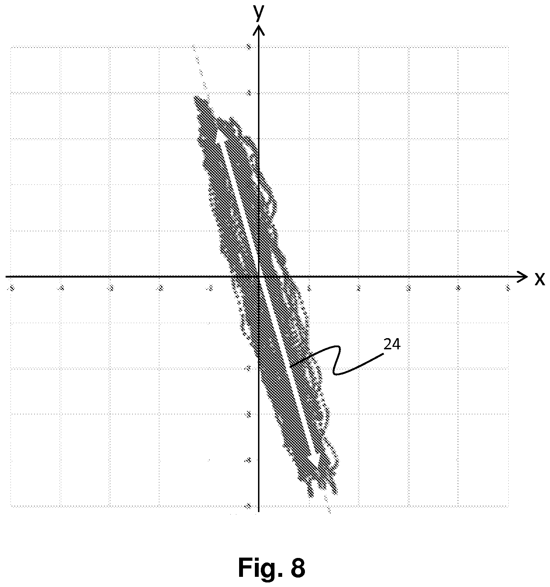

La figure 8 représente schématiquement le mouvement de la référence patient 21 pendant une période de temps comprenant plusieurs cycles de respiration du patient. Le mouvement est représenté dans un système à deux coordonnées x et y. Chaque axe x ou y correspond ainsi à une composante du mouvement de la référence patient 21.

Par exemple, pendant la période de temps considérée, une ligne de mire directe est toujours disponible entre la référence patient 21 et les capteurs optiques 41 du dispositif de localisation 40. La position de chaque marqueur optique 26 de la référence patient 21 est déterminée à plusieurs instants au cours de la période de temps. Il est alors possible de déterminer, pour la période de temps considérée, un mouvement estimé 24 correspondant par exemple au mouvement moyen de la référence patient 21, sur les deux composantes choisies, au cours d'un cycle de respiration du patient. Dans la suite de la description, on considère pour simplifier que le mouvement estimé 24 de la référence patient 21 est sensiblement identique au mouvement suivi par chaque marqueur optique 26 de la référence patient 21. En réalité, chaque marqueur optique 26 de la référence patient 21 pourrait avoir un mouvement particulier, et le mouvement de la référence patient correspondrait alors à une résultante des mouvements des différents capteurs optiques (il serait alors envisageable de s'intéresser à un mouvement estimé pour chaque marqueur optique 26 de la référence patient 21).

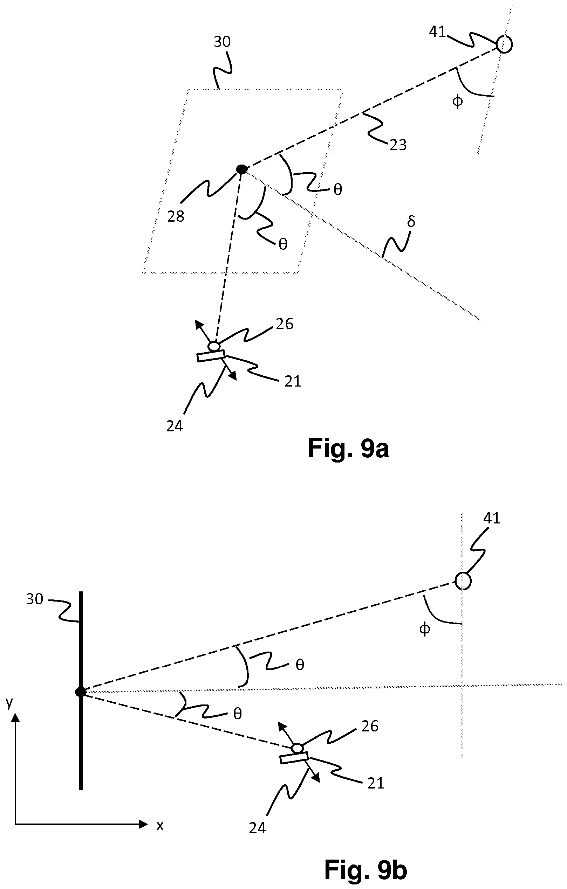

Les figures 9a et 9b représentent schématiquement comment la position d'un marqueur optique 26 de la référence patient 21 peut être déterminée en fonction du mouvement 24 estimé dudit marqueur optique 26 lorsque la ligne de mire est coupée par un obstacle. La figure 9b est une représentation en deux dimensions de la figure 9a dans le plan contenant le rayon infrarouge 23 provenant du marqueur optique 26 et réfléchi par le dispositif réfléchissant 30 ainsi que la droite δ perpendiculaire au dispositif réfléchissant 30 passant par le point de réflexion 28.

Tel qu'illustré sur les figures 9a et 9b, lorsque la ligne de mire n'est plus disponible entre la référence patient 21 et un capteur optique 41, il est possible de déterminer la position d'un marqueur optique 26 en fonction d'une part des mesures réalisées par un capteur optique 41 à partir du rayon optique 23 provenant dudit marqueur optique 26 de la référence patient 21 et réfléchi par le dispositif réfléchissant 30, et en fonction d'autre part du mouvement 24 estimé du marqueur optique 26. Tel qu'illustré sur la figure 9b, le mouvement estimé 24 du marqueur optique 26 est décomposé selon deux composantes orthogonales x et y appartenant au plan contenant le rayon infrarouge 23 et la droite δ. Le rayon infrarouge 23 se réfléchit dans le dispositif réfléchissant 30 au niveau du point de réflexion 28 en formant un angle de réflexion θ par rapport à la droite δ. Cet angle de réflexion θ est identique pour le rayon incident et pour le rayon réfléchi. Le rayon infrarouge 23 forme au niveau du capteur optique 41 un angle d'arrivée Φ.

Si l'angle d'arrivée Φ au niveau du capteur optique 41 et la position du dispositif réfléchissant 30 sont connues, il est possible de déterminer la valeur de l'angle de réflexion θ. Il est alors possible de déterminer la position du marqueur optique 26 car elle correspond à l'intersection du rayon infrarouge 23 avec la trajectoire suivie par le mouvement estimé 24 du marqueur optique 26.

Selon un autre exemple, si le temps de trajet du rayon infrarouge 23 entre le marqueur optique 26 et la capteur optique 41 est connu, autrement dit si la distance parcourue par ledit rayon infrarouge est connue, il est également possible de déterminer la position du marqueur optique 26 car il n'existe qu'un seul point sur la trajectoire suivie par le mouvement estimé 24 du marqueur optique 26 à partir duquel un rayon infrarouge 23 se réfléchissant sur le dispositif réfléchissant 30 atteindrait le capteur optique 41 en parcourant ladite distance.

Dès que la position de chaque marqueur optique 26 est déterminée, la position de la référence patient 21 peut elle aussi être déterminée. Il est ensuite possible d'en déduire la position de l'anatomie d'intérêt, notamment si la position de l'anatomie d'intérêt peut être définie par rapport à la position de la référence patient sur une image médicale.

Il est en outre possible d'utiliser un modèle biomécanique de l'anatomie d'intérêt afin d'optimiser la détermination de la position de l'anatomie d'intérêt en fonction de la position de la référence patient 21 et du mouvement estimé 24 suivi par la référence patient 21. Il est en effet possible de modéliser sous la forme d'un modèle biomécanique les déformations des différentes structures anatomiques (muscles, tendons, structures osseuses, organes, réseau vasculaire, etc.) et les interactions mécaniques entre ces différentes structures. Le modèle biomécanique peut alors permettre de mieux définir le mouvement suivi par l'anatomie d'intérêt en fonction du mouvement suivi par la référence patient 21.

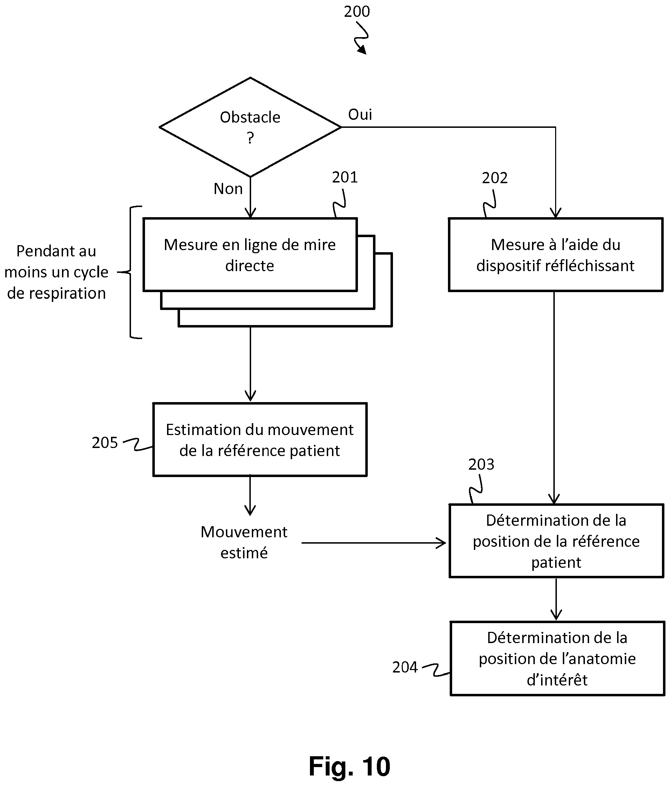

La figure 10 représente schématiquement un mode particulier de mise en oeuvre du procédé 200 décrit en référence à la figure 7. Notamment, l'étape 201 de mesure en ligne de mire directe de la position des marqueurs optiques 26 de la référence patient est répétée à plusieurs instants différents pendant une période de temps comportant au moins un cycle de respiration du patient. Comme cela a été décrit précédemment en référence à la figures 8, cela permet, à l'étape 205, d'estimer un mouvement de la référence patient 21 au cours d'un cycle de respiration du patient. Ce mouvement estimé 24 est alors utilisé à l'étape 203 pour déterminer la position de la référence patient 21 lorsque la ligne de mire n'est plus disponible (comme cela a été décrit précédemment en référence aux figures 9a et 9b). La position de l'anatomie d'intérêt du patient peut ensuite être déterminée, à l'étape 204, à partir de la position de la référence patient 21.

L'invention permet ainsi de déterminer la position d'une anatomie d'intérêt d'un patient à l'aide d'un système de navigation optique 100 même lorsqu'une ligne de mire directe n'est pas disponible entre la référence patient 21 et les capteurs optiques 41 d'un dispositif de localisation 40 du système de navigation optique 100.

Dans des modes particuliers de réalisation, et tel qu'illustré sur les figures 1 et 2, le dispositif de localisation 40 peut en outre comporter une caméra 42 qui fournit des images à l'unité de contrôle 43 pour déterminer une profondeur d'insertion de l'instrument médical pendant l'intervention. Des images de l'intervention sont prises en continu par la caméra 42. Lorsque le robot médical 10 est positionné par rapport au patient conformément au plan d'intervention, le praticien insère l'instrument médical (par exemple une aiguille) dans l'anatomie d'intérêt du patient en vue d'atteindre une zone cible (par exemple une tumeur). Les images de la caméra 42 sont analysées par l'unité de contrôlé 43 qui est configurée pour détecter l'instrument médical. A partir de la connaissance de la longueur totale de l'instrument médical et de la position de la zone cible à atteindre, l'unité de contrôle 43 peut déterminer la profondeur d'insertion de l'instrument médical et déterminer quand l'instrument a atteint la zone cible. Lorsque la zone cible est atteinte, un message à l'attention du praticien s'affiche par exemple sur un écran de contrôle. Le message peut être accompagné d'un signal sonore.

Dans des modes particuliers de réalisation, les images fournies par la caméra 42 du dispositif de localisation 40 sont analysées par l'unité de contrôle 43 pour reconnaître des gestes effectuées par une main (gantée ou non) du praticien, lesdits gestes étant associés à des commandes particulières destinées au robot médical 10 (par exemple pour configurer le bras articulé du robot médical dans une position prédéterminée, pour déplacer la base du robot médical à une position prédéterminée, pour interrompre en urgence tout mouvement du robot médical, etc.). Les gestes effectués par le praticien peuvent obstruer la ligne de mire entre la référence patient 21 et les capteurs optiques 41, le dispositif réfléchissant 30 permet néanmoins au système de navigation optique 100 selon l'invention de déterminer la position de la référence patient 21.

Il convient de noter que plusieurs dispositifs réfléchissants 30 peuvent être utilisés pour augmenter le nombre de trajets différents suivis par un rayonnement optique provenant d'un marqueur optique. Dans des modes particuliers de réalisation, le système de navigation optique 100 comporte avantageusement trois dispositifs réfléchissants différents agencés orthogonalement deux à deux. Quand une ligne de mire directe n'est pas disponible, la position de la référence patient 21 peut alors être déterminée à partir de rayons optiques réfléchis par l'un ou plusieurs des dispositifs réfléchissants.

## Revendications

1. Système de navigation optique (100) pour déterminer la position d'une anatomie d'intérêt d'un patient (20), comprenant :
- un dispositif de localisation (40) comportant au moins deux capteurs optiques (41),
- une unité de contrôle (43),
- une référence patient (21) comportant au moins trois marqueurs optiques (26), les positions respectives des marqueurs optiques (26) les uns par rapport aux autres étant connues a priori par l'unité de contrôle (43), ladite référence patient étant destinée à être positionnée sur le patient (20) au niveau de l'anatomie d'intérêt,
ledit système de navigation optique (100) étant **caractérisé en ce que** :
- le système de navigation optique (100) comporte en outre un dispositif réfléchissant (30) dont la position dans un référentiel du dispositif de localisation (40) est déterminable par l'unité de contrôle (43),
- quand une ligne de mire directe entre la référence patient (21) et chaque capteur optique (41) est disponible, les capteurs optiques (41) sont configurés pour mesurer, pour chaque marqueur optique (26) de la référence patient (21), une grandeur représentative de la position dudit marqueur optique (26) dans le référentiel du dispositif de localisation (40) à partir d'un rayonnement optique provenant dudit marqueur optique (26) et présentant pour chaque capteur optique (41) un trajet direct (22) entre ledit marqueur optique (26) et ledit capteur optique (41),
- quand une ligne de mire directe entre la référence patient (21) et un capteur optique (41) est coupée par un obstacle (60), les capteurs optiques (41) sont configurés pour mesurer, pour chaque marqueur optique (26) de la référence patient (21), une grandeur représentative de la position dudit marqueur optique (26) dans le référentiel du dispositif de localisation (40) à partir d'un rayonnement optique provenant dudit marqueur optique (26) et présentant un trajet réfléchi (23) par le dispositif réfléchissant (30) vers chaque capteur optique (41),
- l'unité de contrôle (43) est configurée pour déterminer, à partir des mesures réalisées par les capteurs optiques (41), la position de la référence patient (21) dans le référentiel du dispositif de localisation (40), et pour en déduire la position de l'anatomie d'intérêt dans ledit référentiel.

2. Système de navigation optique (100) selon la revendication 1 dans lequel le dispositif réfléchissant (30) comporte au moins trois marqueurs optiques (36), les positions respectives des marqueurs optiques (36) les uns par rapport aux autres étant connues a priori par l'unité de contrôle (43).

3. Système de navigation optique (100) selon l'une des revendications 1 à 2 dans lequel l'unité de contrôle (43) est configurée pour :
- pendant une période où une ligne de mire directe entre la référence patient (21) et chaque capteur optique (41) est disponible, estimer un mouvement (24) suivi par la référence patient (21) dans le référentiel du dispositif de localisation (40) pendant un cycle de respiration du patient (20),
- à un instant où une ligne de mire directe entre la référence patient (21) et un capteur optique (41) n'est plus disponible, déterminer la position de la référence patient (21) en fonction d'une part des mesures réalisées par les capteurs optiques (41) à partir des rayonnements optiques provenant des marqueurs optiques (26) de la référence patient (21) et réfléchis par le dispositif réfléchissant (30), et en fonction d'autre part du mouvement (24) estimé de la référence patient (21).

4. Système de navigation optique (100) selon l'une des revendications 1 à 3 dans lequel la référence patient (21) comporte en outre au moins trois marqueurs radio-opaques (27), les positions respectives des marqueurs radio-opaques (27) les uns par rapport aux autres étant connues a priori par l'unité de contrôle (43).

5. Système de navigation optique (100) selon la revendication 4 dans lequel la position de l'anatomie d'intérêt dans le référentiel du dispositif de localisation (40) est déterminée en fonction de la position de la référence patient (21) dans ledit référentiel et en fonction d'une image médicale de l'anatomie d'intérêt du patient sur laquelle sont visibles les marqueurs radio-opaques (27) de la référence patient (21).

6. Système de navigation optique (100) selon la revendication 5 dans lequel la position de l'anatomie d'intérêt dans le référentiel du dispositif de localisation (40) est déterminée en outre en fonction d'un modèle biomécanique de l'anatomie d'intérêt.

7. Système de navigation optique (100) selon l'une des revendications 1 à 6 comportant trois dispositifs réfléchissants (30) orthogonaux deux à deux.

8. Système de navigation optique (100) selon l'une des revendications 1 à 7 comportant en outre une référence robot (11) destinée à être positionnée au niveau d'une extrémité distale d'un bras articulé (13) d'un robot médical (10), ladite référence robot (11) comportant au moins trois marqueurs optiques (16), les positions respectives des marqueurs optiques (16) les uns par rapport aux autres étant connues a priori par l'unité de contrôle (43), et dans lequel
- quand une ligne de mire directe entre la référence robot (11) et chaque capteur optique (41) est disponible les capteurs optiques (41) sont configurés pour mesurer, pour chaque marqueur optique (16) de la référence robot (11), une grandeur représentative de la position dudit marqueur optique (16) dans le référentiel du dispositif de localisation (40), à partir d'un rayonnement optique provenant dudit marqueur optique (16) et présentant pour chaque capteur optique un trajet direct entre ledit marqueur optique et ledit capteur optique (41),
- quand une ligne de mire directe entre la référence robot (11) et un capteur optique (41) est coupée par un obstacle, les capteurs optiques (41) sont configurés pour mesurer, pour chaque marqueur optique (16) de la référence robot (11), une grandeur représentative de la position dudit marqueur optique (16) dans le référentiel du dispositif de localisation (40) à partir d'un rayonnement optique provenant dudit marqueur optique (16) et présentant un trajet réfléchi par le dispositif réfléchissant (30) vers chaque capteur optique (41),
- l'unité de contrôle (43) est configurée pour déterminer la position de la référence robot (11) dans le référentiel du dispositif de localisation (40) à partir des mesures ainsi réalisées par les capteurs optiques (41).

9. Système de navigation optique (100) selon la revendication 8 comportant en outre un robot médical (10) comportant un bras articulé (13), la référence robot (11) étant positionnée à une extrémité distale du bras articulé (13), ledit robot médical (10) comportant en outre des codeurs d'articulation du bras articulé (13) permettant de déterminer à tout instant la position de la référence robot (11) dans un référentiel du robot médical (10), le robot médical (10) étant configuré pour transmettre à l'unité de contrôle (43) la position de la référence robot (11) dans le référentiel du robot médical (10), et l'unité de contrôle (43) est configurée pour en déduire la position d'un instrument médical fixé à l'extrémité distale du bras articulé (13) du robot médical (10) par rapport à l'anatomie d'intérêt du patient (20).

10. Système de navigation optique (100) selon l'une des revendications 8 à 9 dans lequel les marqueurs optiques (26, 16) de la référence patient (21) et/ou de la référence robot (11) sont des marqueurs actifs et le rayonnement optique provenant d'un marqueur optique (26, 16) est un rayonnement infrarouge généré par ledit marqueur optique (26, 16).

11. Système de navigation optique (100) selon l'une des revendications 8 à 9 dans lequel les marqueurs optiques (26, 16) de la référence patient (21) et/ou de la référence robot (11) sont des marqueurs passifs et le rayonnement optique provenant d'un marqueur optique (26, 16) est un rayonnement infrarouge généré par le dispositif de localisation (40) et réfléchi par ledit marqueur optique (26, 16).

12. Procédé (200) pour déterminer la position d'une anatomie d'intérêt d'un patient (20), ledit procédé (200) étant mis en oeuvre par un système de navigation optique (100) comprenant :
- un dispositif de localisation (40) comportant au moins deux capteurs optiques (41),
- une référence patient (21) comportant au moins trois marqueurs optiques (26), les positions respectives des marqueurs optiques (26) les uns par rapport aux autres étant connues a priori, ladite référence patient (21) étant destinée à être positionnée sur le patient (20) au niveau de l'anatomie d'intérêt,
ledit procédé étant **caractérisé en ce que** le système de navigation optique (100) comporte en outre un dispositif réfléchissant (30) dont la position dans un référentiel du dispositif de localisation (40) est connue, et le procédé (200) comporte les étapes suivantes :
- quand une ligne de mire directe entre la référence patient (21) et chaque capteur optique (41) est disponible, une mesure (201), pour chaque marqueur optique (26) de la référence patient (21), d'une grandeur représentative de la position dudit marqueur optique (26) dans le référentiel du dispositif de localisation (40) à partir d'un rayonnement optique provenant dudit marqueur optique (26) et présentant pour chaque capteur optique (41) un trajet direct (22) entre ledit marqueur optique (26) et ledit capteur optique (41),
- quand une ligne de mire directe entre la référence patient (21) et un capteur optique (41) est coupée par un obstacle (60), une mesure (202), pour chaque marqueur optique (26) de la référence patient (20), d'une grandeur représentative de la position dudit marqueur optique (26) dans le référentiel du dispositif de localisation (40) à partir d'un rayonnement optique provenant dudit marqueur optique (26) et présentant un trajet réfléchi (23) par le dispositif réfléchissant (30) vers chaque capteur optique (41),
- une détermination (203), à partir des mesures ainsi réalisées par les capteurs optiques (41), de la position de la référence patient (21) dans le référentiel du dispositif de localisation (40),
- une détermination (204), dans ledit référentiel du dispositif de localisation (40), de la position de l'anatomie d'intérêt à partir de la position de la référence patient (21).

13. Procédé (200) selon la revendication 12 comportant en outre les étapes suivantes :
- pendant une période où une ligne de mire directe entre la référence patient (21) et chaque capteur optique (41) est disponible, une estimation (205) d'un mouvement (24) suivi par la référence patient (21) dans le référentiel du dispositif de localisation (40) pendant un cycle de respiration du patient (20),
- à un instant où une ligne de mire directe entre la référence patient (21) et un capteur optique (41) n'est plus disponible, une détermination (203) de la position de la référence patient (21) en fonction d'une part des mesures réalisées par les capteurs optiques (41) à partir des rayonnements optiques provenant des marqueurs optiques (26) de la référence patient (21) et réfléchis par le dispositif réfléchissant (30), et en fonction d'autre part du mouvement (24) estimé de la référence patient (21).

14. Procédé (200) selon la revendication 13 dans lequel la détermination (204) de la position de l'anatomie d'intérêt dans le référentiel du dispositif de localisation (40) est effectuée en outre à partir d'une image médicale de l'anatomie d'intérêt du patient (20) sur laquelle sont visibles des marqueurs radio-opaques (27) de la référence patient (21).

15. Procédé (200) selon la revendication 14 dans lequel la détermination (204) de la position de l'anatomie d'intérêt dans le référentiel du dispositif de localisation (40) est effectuée en outre à partir d'un modèle biomécanique de l'anatomie d'intérêt.

16. Procédé (200) selon l'une des revendications 12 à 15 dans lequel le système de navigation optique (100) comporte en outre une référence robot (11) destinée à être positionnée au niveau d'une extrémité distale d'un bras articulé (13) d'un robot médical (10), ladite référence robot (11) comportant au moins trois marqueurs optiques (16), les positions respectives des marqueurs optiques (16) les uns par rapport aux autres étant connues a priori, ledit procédé (200) comportant en outre les étapes suivantes :
- quand une ligne de mire directe entre la référence robot (11) et chaque capteur optique (41) est disponible, une mesure, pour chaque marqueur optique (16) de la référence robot (11), d'une grandeur représentative de la position dudit marqueur optique (16) dans le référentiel du dispositif de localisation (40), à partir d'un rayonnement optique provenant dudit marqueur optique (16) et présentant pour chaque capteur optique (41) un trajet direct entre ledit marqueur optique (16) et ledit capteur optique (41),
- quand une ligne de mire directe entre la référence robot (11) et un capteur optique (41) est coupée par un obstacle, une mesure, pour chaque marqueur optique (16) de la référence robot (11), d'une grandeur représentative de la position dudit marqueur optique (16) dans le référentiel du dispositif de localisation (40) à partir d'un rayonnement optique provenant dudit marqueur optique (16) et présentant un trajet réfléchi par le dispositif réfléchissant (30) vers chaque capteur optique (41),
- une détermination de la position de la référence robot (11) dans le référentiel du dispositif de localisation (40) à partir des mesures ainsi réalisées par les capteurs optiques (41).

17. Procédé (200) selon la revendication 16 dans lequel le système de navigation optique (100) comporte en outre en outre un robot médical (10) comportant un bras articulé (13), la référence robot (11) étant positionnée à une extrémité distale du bras articulé (13), ledit robot médical (10) comportant en outre des codeurs d'articulation du bras articulé (13) permettant de déterminer à tout instant la position de la référence robot (11) dans un référentiel du robot médical (10), ledit procédé (200) comportant en outre une étape de détermination de la position d'un instrument médical fixé à l'extrémité distale du bras articulé (13) du robot médical (10) par rapport à l'anatomie d'intérêt du patient (20).

## Patentansprüche

1. Optisches Navigationssystem (100) zur Bestimmung der Position einer Anatomie von Interesse eines Patienten (20), umfassend:
- eine Lokalisierungsvorrichtung (40), umfassend mindestens zwei optische Sensoren (41),
- eine Steuereinheit (43),
- eine Patientenreferenz (21), umfassend mindestens drei optische Marker (26), wobei die jeweiligen Positionen der optischen Marker (26) zueinander durch die Steuereinheit (43) a priori bekannt sind, wobei die Patientenreferenz dazu bestimmt ist, am Patienten (20) im Bereich der Anatomie von Interesse positioniert zu werden,
wobei das optische Navigationssystem (100)
**dadurch gekennzeichnet ist, dass:**
- das optische Navigationssystem (100) ferner eine reflektierende Vorrichtung (30) umfasst, deren Position in einem Bezugssystem der Lokalisierungsvorrichtung (40) durch die Steuereinheit (43) bestimmbar ist,
- wenn eine direkte Sichtlinie zwischen der Patientenreferenz (21) und jedem optischen Sensor (41) verfügbar ist, die optischen Sensoren (41) so konfiguriert sind, dass sie für jeden optischen Marker (26) der Patientenreferenz (21) eine Größe messen, welche die Position des optischen Markers (26) im Bezugssystem der Lokalisierungsvorrichtung (40) anhand einer optischen Strahlung darstellt, die von dem optischen Marker (26) ausgeht und für jeden optischen Sensor (41) einen direkten Pfad (22) zwischen dem optischen Marker (26) und dem optischen Sensor (41) aufweist,
- wenn eine direkte Sichtlinie zwischen der Patientenreferenz (21) und einem optischen Sensor (41) durch ein Hindernis (60) unterbrochen wird, die optischen Sensoren (41) so konfiguriert sind, dass sie für jeden optischen Marker (26) der Patientenreferenz (21) eine Größe messen, welche die Position des optischen Markers (26) im Bezugssystem der Lokalisierungsvorrichtung (40) anhand einer optischen Strahlung darstellt, die von dem optischen Marker (26) ausgeht und einen reflektierten Pfad (23) durch die reflektierende Vorrichtung (30) zu jedem optischen Sensor (41) aufweist,
- die Steuereinheit (43) konfiguriert ist, um anhand der durch die optischen Sensoren (41) durchgeführten Messungen die Position der Patientenreferenz (21) im Bezugssystem der Lokalisierungsvorrichtung (40) zu bestimmen und daraus die Position der Anatomie von Interesse in diesem Bezugssystem abzuleiten.

2. Optisches Navigationssystem (100) nach Anspruch 1, bei dem die reflektierende Vorrichtung (30) mindestens drei optische Marker (36) umfasst, wobei die jeweiligen Positionen der optischen Marker (36) relativ zueinander durch die Steuereinheit a priori bekannt sind (43).

3. Optisches Navigationssystem (100) nach einem der Ansprüche 1 bis 2, bei dem die Steuereinheit (43) konfiguriert ist zum:
- Schätzen, während eines Zeitraums, in dem eine direkte Sichtlinie zwischen der Patientenreferenz (21) und jedem optischen Sensor (41) verfügbar ist, einer Bewegung (24), der die Patientenreferenz (21) im Referenzsystem der Lokalisierungsvorrichtung (40) während eines Atemzyklus des Patienten (20) folgt,
- Bestimmen, zu einem Zeitpunkt, zu dem eine direkte Sichtverbindung zwischen der Patientenreferenz (21) und einem optischen Sensor (41) nicht mehr verfügbar ist, der Position der Patientenreferenz (21) in Abhängigkeit einerseits von den Messungen, die von den optischen Sensoren (41) anhand der optischen Strahlung durchgeführt werden, die von den optischen Markern (26) der Patientenreferenz (21) ausgeht und von der reflektierenden Vorrichtung (30) reflektiert wird, und in Abhängigkeit andererseits von der geschätzten Bewegung (24) der Patientenreferenz (21).

4. Optisches Navigationssystem (100) nach einem der Ansprüche 1 bis 3, bei dem die Patientenreferenz (21) ferner mindestens drei radioopake Marker (27) umfasst, wobei die jeweiligen Positionen der radioopaken Marker (27) relativ zueinander durch die Steuereinheit (43) a priori bekannt sind.

5. Optisches Navigationssystem (100) nach Anspruch 4, bei dem die Position der Anatomie von Interesse im Referenzsystem der Lokalisierungsvorrichtung (40) in Abhängigkeit von der Position der Patientenreferenz (21) im Referenzsystem und in Abhängigkeit von einem medizinischen Bild der Anatomie von Interesse des Patienten, auf dem die radioopaken Marker (27) der Patientenreferenz (21) sichtbar sind, bestimmt wird.

6. Optisches Navigationssystem (100) nach Anspruch 5, bei dem die Position der Anatomie von Interesse im Bezugssystem der Lokalisierungsvorrichtung (40) ferner in Abhängigkeit von einem biomechanischen Modell der Anatomie von Interesse bestimmt wird.

7. Optisches Navigationssystem (100) nach einem der Ansprüche 1 bis 6, umfassend drei paarweise angeordnete orthogonale Reflexionsvorrichtungen (30).

8. Optisches Navigationssystem (100) nach einem der Ansprüche 1 bis 7, ferner umfassend eine Roboterreferenz (11), die dazu bestimmt ist, auf Höhe eines distalen Endes eines Gelenkarms (13) eines medizinischen Roboters (10) positioniert zu werden, wobei die Roboterreferenz (11) mindestens drei optische Marker (16) umfasst, wobei die jeweiligen Positionen der optischen Marker (16) zueinander a priori durch die Steuereinheit (43) bekannt sind, und bei dem,
- wenn eine direkte Sichtlinie zwischen der Roboterreferenz (11) und jedem optischen Sensor (41) verfügbar ist, die optischen Sensoren (41) so konfiguriert sind, dass sie für jeden optischen Marker (16) der Roboterreferenz (11) eine Größe messen, welche die Position des optischen Markers (16) im Bezugssystem der Lokalisierungsvorrichtung (40) anhand einer optischen Strahlung darstellt, die von dem optischen Marker (16) ausgeht und für jeden optischen Sensor einen direkten Pfad zwischen dem optischen Marker und dem optischen Sensor (41) aufweist,
- wenn eine direkte Sichtlinie zwischen der Roboterreferenz (11) und einem optischen Sensor (41) durch ein Hindernis unterbrochen wird, die optischen Sensoren (41) so konfiguriert sind, dass sie für jeden optischen Marker (16) der Roboterreferenz (11) eine Größe messen, welche die Position des optischen Markers (16) im Bezugssystem der Lokalisierungsvorrichtung (40) anhand einer optischen Strahlung darstellt, die von dem optischen Marker (16) ausgeht und einen reflektierten Pfad durch die reflektierende Vorrichtung (30) zu jedem optischen Sensor (41) aufweist,
- die Steuereinheit (43) konfiguriert ist, um die Position der Roboterreferenz (11) im Bezugssystem der Lokalisierungsvorrichtung (40) anhand der so von den optischen Sensoren (41) durchgeführten Messungen zu bestimmen.

9. Optisches Navigationssystem (100) nach Anspruch 8, ferner umfassend einen medizinischen Roboter (10), der einen Gelenkarm (13) umfasst, wobei die Roboterreferenz (11) an einem distalen Ende des Gelenkarms (13) positioniert ist, wobei der medizinische Roboter (10) ferner Gelenkcodierer des Gelenkarms (13) umfasst, die es ermöglichen, zu jedem Zeitpunkt die Position der Roboterreferenz (11) in einem Bezugssystem des medizinischen Roboters (10) zu bestimmen, wobei der medizinische Roboter (10) konfiguriert ist, um an die Steuereinheit (43) die Position der Roboterreferenz (11) im Bezugssystem des medizinischen Roboters (10) zu übertragen, und die Steuereinheit (43) konfiguriert ist, um daraus die Position eines am distalen Ende des Gelenkarms (13) des medizinischen Roboters (10) befestigten medizinischen Instruments relativ zur Anatomie von Interesse des Patienten (20) abzuleiten.

10. Optisches Navigationssystem (100) nach einem der Ansprüche 8 bis 9, bei dem die optischen Marker (26, 16) der Patientenreferenz (21) und/oder der Roboterreferenz (11) aktive Marker sind und die optische Strahlung, die von einem optischen Marker (26, 16) ausgeht, eine Infrarotstrahlung ist, die von dem optischen Marker (26, 16) erzeugt wird.

11. Optisches Navigationssystem (100) nach einem der Ansprüche 8 bis 9, bei dem die optischen Marker (26, 16) der Patientenreferenz (21) und/oder der Roboterreferenz (11) passive Marker sind und die optische Strahlung, die von einem optischen Marker (26, 16) ausgeht, eine Infrarotstrahlung ist, die von der Lokalisierungsvorrichtung (40) erzeugt und von dem optischen Marker (26, 16) reflektiert wird.

12. Verfahren (200) zum Bestimmen der Position einer Anatomie von Interesse eines Patienten (20), wobei das Verfahren (200) durch ein optisches Navigationssystem (100) implementiert wird, umfassend:
- eine Lokalisierungsvorrichtung (40), umfassend mindestens zwei optische Sensoren (41),
- eine Patientenreferenz (21), die mindestens drei optische Marker (26) umfasst, wobei die jeweiligen Positionen der optischen Marker (26) relativ zueinander a priori bekannt sind, wobei die Patientenreferenz (21) dazu bestimmt ist, am Patienten (20) auf Höhe der Anatomie von Interesse positioniert zu werden,
wobei das Verfahren **dadurch gekennzeichnet ist, dass** das optische Navigationssystem (100) ferner eine reflektierende Vorrichtung (30) umfasst, deren Position in einem Bezugssystem der Lokalisierungsvorrichtung (40) bekannt ist, und das Verfahren (200) die folgenden Schritte umfasst:
- wenn eine direkte Sichtlinie zwischen der Patientenreferenz (21) und jedem optischen Sensor (41) verfügbar ist, eine Messung (201), für jeden optischen Marker (26) der Patientenreferenz (21) einer Größe, welche die Position des optischen Markers (26) im Bezugssystem der Lokalisierungsvorrichtung (40) anhand einer optischen Strahlung darstellt, die von dem optischen Marker (26) ausgeht und für jeden optischen Sensor (41) einen direkten Pfad (22) zwischen dem optischen Marker (26) und dem optischen Sensor (41) aufweist,
- wenn eine direkte Sichtlinie zwischen der Patientenreferenz (21) und einem optischen Sensor (41) durch ein Hindernis (60) unterbrochen wird, eine Messung (202), für jeden optischen Marker (26) der Patientenreferenz (20), einer Größe, welche die Position des optischen Markers (26) im Bezugssystem der Lokalisierungsvorrichtung (40) anhand einer optischen Strahlung darstellt, die von dem optischen Marker (26) ausgeht und einen reflektierten Pfad (23) durch die reflektierende Vorrichtung (30) zu jedem optischen Sensor (41) aufweist,
- eine Bestimmung (203) der Position der Patientenreferenz (21) im Bezugssystem der Lokalisierungsvorrichtung (40) anhand der so durchgeführten Messungen der optischen Sensoren (41),
- eine Bestimmung (204) der Position der Anatomie von Interesse anhand der Position der Patientenreferenz (21) im Referenzsystem der Lokalisierungsvorrichtung (40).

13. Verfahren (200) nach Anspruch 12, ferner umfassend die folgenden Schritte:
- während eines Zeitraums, bei dem eine direkte Sichtlinie zwischen der Patientenreferenz (21) und jedem optischen Sensor (41) verfügbar ist, eine Schätzung (205) einer Bewegung (24), der die Patientenreferenz (21) im Referenzsystem der Lokalisierungsvorrichtung (40) während eines Atemzyklus des Patienten (20) folgt,
- zu einem Zeitpunkt, zu dem eine direkte Sichtverbindung zwischen der Patientenreferenz (21) und einem optischen Sensor (41) nicht mehr verfügbar ist, eine Bestimmung (203) der Position der Patientenreferenz (21) in Abhängigkeit einerseits von den Messungen, die von den optischen Sensoren (41) anhand der optischen Strahlung durchgeführt werden, die von den optischen Markern (26) der Patientenreferenz (21) ausgeht und von der reflektierenden Vorrichtung (30) reflektiert wird, und in Abhängigkeit andererseits von der geschätzten Bewegung (24) der Patientenreferenz (21).

14. Verfahren (200) nach Anspruch 13, bei dem die Bestimmung (204) der Position der Anatomie von Interesse im Referenzsystem der Lokalisierungsvorrichtung (40) ferner anhand eines medizinischen Bildes der Anatomie von Interesse des Patienten erfolgt (20), auf dem radioopake Marker (27) der Patientenreferenz (21) sichtbar sind.

15. Verfahren (200) nach Anspruch 14, bei dem die Bestimmung (204) der Position der Anatomie von Interesse im Bezugssystem der Lokalisierungsvorrichtung (40) ferner anhand eines biomechanischen Modells der Anatomie von Interesse erfolgt.

16. Verfahren (200) nach einem der Ansprüche 12 bis 15, bei dem das optische Navigationssystem (100) ferner eine Roboterreferenz (11) umfasst, die dazu bestimmt ist, auf Höhe eines distalen Endes eines Gelenkarms (13) eines medizinischen Roboters positioniert zu werden (10), wobei die Roboterreferenz (11) mindestens drei optische Marker (16) umfasst, wobei die jeweiligen Positionen der optischen Marker (16) relativ zueinander a priori bekannt sind, wobei das Verfahren (200) ferner die folgenden Schritte umfasst:
- wenn eine direkte Sichtlinie zwischen der Roboterreferenz (11) und jedem optischen Sensor (41) verfügbar ist, eine Messung, für jeden optischen Marker (16) der Roboterreferenz (11), einer Größe, welche die Position des optischen Markers (16) im Bezugssystem der Lokalisierungsvorrichtung (40) anhand einer optischen Strahlung darstellt, die von dem optischen Marker (16) ausgeht und für jeden optischen Sensor (41) einen direkten Pfad zwischen dem optischen Marker (16) und dem optischen Sensor (41) aufweist,
- wenn eine direkte Sichtlinie zwischen der Roboterreferenz (11) und einem optischen Sensor (41) durch ein Hindernis unterbrochen wird, eine Messung, für jeden optischen Marker (16) der Roboterreferenz (11), einer Größe, welche die Position des optischen Markers (16) im Bezugssystem der Lokalisierungsvorrichtung (40) anhand einer optischen Strahlung darstellt, die von dem optischen Marker (16) ausgeht und einen reflektierten Pfad durch die reflektierende Vorrichtung (30) zu jedem optischen Sensor (41) aufweist,
- eine Bestimmung der Position der Roboterreferenz (11) im Bezugssystem der Lokalisierungsvorrichtung (40) anhand der so durchgeführten Messungen durch die optischen Sensoren (41).

17. Verfahren (200) nach Anspruch 16, bei dem das optische Navigationssystem (100) ferner einen medizinischen Roboter (10) umfasst, der einen Gelenkarm (13) umfasst, wobei die Roboterreferenz (11) an einem distalen Ende des Gelenkarms (13) positioniert ist, wobei der medizinische Roboter (10) ferner Gelenkcodierer des Gelenkarms (13) umfasst, die es ermöglichen, zu jedem Zeitpunkt die Position der Roboterreferenz (11) in einem Bezugssystem des medizinischen Roboters (10) zu bestimmen, wobei das Verfahren (200) ferner einen Schritt des Bestimmens der Position eines am distalen Ende des Gelenkarms (13) des medizinischen Roboters (10) befestigten medizinischen Instruments in Bezug auf die Anatomie von Interesse des Patienten umfasst (20).

## Claims

1. Optical navigation system (100) for determining the position of a patient's anatomy of interest (20), comprising :
- a locating device (40) with at least two optical sensors (41),
- a control unit (43),
- a patient reference (21) comprising at least three optical markers (26), the respective positions of the optical markers (26) relative to one another being known a priori by the control unit (43), said patient reference being intended to be positioned on the patient (20) at the anatomy of interest,
said optical navigation system (100) being **characterized in that** :
- the optical navigation system (100) further comprises a reflective device (30) whose position in a reference frame of the locating device (40) can be determined by the control unit (43),
- when a direct line of sight between the patient reference (21) and each optical sensor (41) is available, the optical sensors (41) are configured to measure, for each optical marker (26) of the patient reference (21), a quantity representative of the position of said optical marker (26) in the reference frame of the locating device (40) from optical radiation originating from said optical marker (26) and having for each optical sensor (41) a direct path (22) between said optical marker (26) and said optical sensor (41),
- when a direct line of sight between the patient reference (21) and an optical sensor (41) is intersected by an obstacle (60), the optical sensors (41) are configured to measure, for each optical marker (26) of the patient reference (21), a quantity representative of the position of said optical marker (26) in the reference frame of the locating device (40) from optical radiation originating from said optical marker (26) and having a path reflected (23) by the reflective device (30) to each optical sensor (41),
- the control unit (43) is configured to determine, from the measurements made by the optical sensors (41), the position of the patient reference (21) in the frame of reference of the locating device (40), and to deduce therefrom the position of the anatomy of interest in said frame of reference.

2. The optical navigation system (100) according to claim 1, wherein the reflective device (30) comprises at least three optical markers (36), the respective positions of the optical markers (36) relative to one another being known a priori by the control unit (43).

3. The optical navigation system (100) according to one of claims 1 to 2, wherein the control unit (43) is configured for:
- estimating a movement (24) followed by the patient reference (21) in the reference frame of the locating device (40) during a patient's breathing cycle (20), during a period when a direct line of sight between the patient reference (21) and each optical sensor (41) is available,
- at a time when a direct line of sight between the patient reference (21) and an optical sensor (41) is no longer available, determining the position of the patient reference (21) as a function on the one hand of the measurements made by the optical sensors (41) from the optical radiation originating from the optical markers (26) of the patient reference (21) and reflected by the reflective device (30), and on the other hand as a function of the estimated movement (24) of the patient reference (21).

4. The optical navigation system (100) according to one of claims 1 to 3, wherein the patient reference (21) further comprises at least three radio-opaque markers (27), the respective positions of the radio-opaque markers (27) relative to one another being known a priori by the control unit (43).

5. The optical navigation system (100) according to claim 4, wherein the position of the anatomy of interest in the reference frame of the locating device (40) is determined as a function of the position of the patient reference (21) in said reference frame and as a function of a medical image of the patient's anatomy of interest on which the radio-opaque markers (27) of the patient reference (21) are visible.

6. The optical navigation system (100) according to claim 5, wherein the position of the anatomy of interest in the frame of reference of the locating device (40) is further determined as a function of a biomechanical model of the anatomy of interest.

7. The optical navigation system (100) according to one of claims 1 to 6, comprising three reflective devices (30) orthogonal in pairs.

8. The optical navigation system (100) according to one of claims 1 to 7, further comprising a robot reference (11) intended to be positioned at a distal end of an articulated arm (13) of a medical robot (10), said robot reference (11) comprising at least three optical markers (16), the respective positions of the optical markers (16) relative to one another being known a priori by the control unit (43), and wherein
- when a direct line of sight between the robot reference (11) and each optical sensor (41) is available, the optical sensors (41) are configured to measure, for each optical marker (16) of the robot reference (11), a quantity representative of the position of said optical marker (16) in the reference frame of the locating device (40), from optical radiation originating from said optical marker (16) and having for each optical sensor a direct path between said optical marker and said optical sensor (41),
- when a direct line of sight between the robot reference (11) and an optical sensor (41) is intersected by an obstacle, the optical sensors (41) are configured to measure, for each optical marker (16) of the robot reference (11), a quantity representative of the position of said optical marker (16) in the reference frame of the locating device (40) from optical radiation originating from said optical marker (16) and having a path reflected by the reflective device (30) towards each optical sensor (41),
- the control unit (43) is configured to determine the position of the robot reference (11) in the reference frame of the locating device (40) from the measurements thus made by the optical sensors (41).

9. The optical navigation system (100) according to claim 8, further comprising a medical robot (10) comprising an articulated arm (13), the robot reference (11) being positioned at a distal end of the articulated arm (13), said medical robot (10) further comprising articulation encoders of the articulated arm (13) enabling the position of the robot reference (11) in a reference frame of the medical robot (10) to be determined at any time, the medical robot (10) being configured to transmit to the control unit (43) the position of the robot reference (11) in the frame of reference of the medical robot (10), and the control unit (43) is configured to deduce therefrom the position of a medical instrument attached to the distal end of the articulated arm (13) of the medical robot (10) relative to the anatomy of interest of the patient (20).

10. The optical navigation system (100) according to one of claims 8 to 9, wherein the optical markers (26, 16) of the patient reference (21) and/or robot reference (11) are active markers and the optical radiation from an optical marker (26, 16) is infrared radiation generated by said optical marker (26, 16).

11. The optical navigation system (100) according to one of claims 8 to 9, wherein the optical markers (26, 16) of the patient reference (21) and/or robot reference (11) are passive markers and the optical radiation from an optical marker (26, 16) is infrared radiation generated by the locating device (40) and reflected by said optical marker (26, 16).

12. A method (200) for determining the position of an anatomy of interest of a patient (20), said method (200) being implemented by an optical navigation system (100) comprising:
- a locating device (40) with at least two optical sensors (41),
- a patient reference (21) comprising at least three optical markers (26), the respective positions of the optical markers (26) relative to one another being known a priori, said patient reference (21) being intended to be positioned on the patient (20) at the anatomy of interest,
said method being **characterized in that** the optical navigation system (100) further comprises a reflective device (30) whose position in a reference frame of the locating device (40) is known, and the method (200) comprises the following steps:
- when a direct line of sight between the patient reference (21) and each optical sensor (41) is available, measuring (201), for each optical marker (26) of the patient reference (21), of a quantity representative of the position of said optical marker (26) in the reference frame of the locating device (40) from optical radiation originating from said optical marker (26) and having for each optical sensor (41) a direct path (22) between said optical marker (26) and said optical sensor (41),
- when a direct line of sight between the patient reference (21) and an optical sensor (41) is intersected by an obstacle (60), measuring (202), for each optical marker (26) of the patient reference (20), of a quantity representative of the position of said optical marker (26) in the reference frame of the locating device (40) from optical radiation originating from said optical marker (26) and having a path reflected (23) by the reflective device (30) to each optical sensor (41),
- determining (203) the position of the patient reference (21) in the reference frame of the locating device (40), based on the measurements made by the optical sensors (41),
- determining (204), in said reference frame of the locating device (40), the position of the anatomy of interest from the position of the patient reference (21).

13. The method (200) according to claim 12, further comprising the following steps:
- during a period when a direct line of sight between the patient reference (21) and each optical sensor (41) is available, estimating (205) a movement (24) followed by the patient reference (21) in the reference frame of the locating device (40) during a breathing cycle of the patient (20),
- at a time when a direct line of sight between the patient reference (21) and an optical sensor (41) is no longer available, determining (203) the position of the patient reference (21) as a function on the one hand of the measurements made by the optical sensors (41) from the optical radiation originating from the optical markers (26) of the patient reference (21) and reflected by the reflective device (30), and as a function on the other hand of the estimated movement (24) of the patient reference (21).

14. The method (200) according to claim 13, wherein the determination (204) of the position of the anatomy of interest in the reference frame of the locating device (40) is further carried out on the basis of a medical image of the patient's anatomy of interest (20) on which radio-opaque markers (27) of the patient reference (21) are visible.

15. The method (200) according to claim 14, wherein the determination (204) of the position of the anatomy of interest in the reference frame of the locating device (40) is additionally carried out from a biomechanical model of the anatomy of interest.

16. The method (200) according to one of claims 12 to 15, wherein the optical navigation system (100) further comprises a robot reference (11) intended to be positioned at a distal end of an articulated arm (13) of a medical robot (10), said robot reference (11) comprising at least three optical markers (16), the respective positions of the optical markers (16) relative to one another being known a priori, said method (200) further comprising the following steps:
- when a direct line of sight between the robot reference (11) and each optical sensor (41) is available, measuring, for each optical marker (16) of the robot reference (11), a quantity representative of the position of said optical marker (16) in the reference frame of the locating device (40), from optical radiation originating from said optical marker (16) and having for each optical sensor (41) a direct path between said optical marker (16) and said optical sensor (41),
- when a direct line of sight between the robot reference (11) and an optical sensor (41) is intersected by an obstacle, measuring, for each optical marker (16) of the robot reference (11), a quantity representative of the position of said optical marker (16) in the reference frame of the locating device (40) from optical radiation originating from said optical marker (16) and having a path reflected by the reflective device (30) towards each optical sensor (41),
- determining the position of the robot reference (11) in the reference frame of the locating device (40) from the measurements thus made by the optical sensors (41).

17. The method (200) according to claim 16, wherein the optical navigation system (100) further comprises a medical robot (10) comprising an articulated arm (13), the robot reference (11) being positioned at a distal end of the articulated arm (13), said medical robot (10) further comprising articulation encoders of the articulated arm (13) making it possible to determine at any time the position of the robot reference (11) in a reference frame of the medical robot (10), said method (200) further comprising a step of determining the position of a medical instrument attached to the distal end of the articulated arm (13) of the medical robot (10) with respect to the anatomy of interest of the patient (20).
